# EUROPÄISCHE PATENTSCHRIFT

(11) **EP 1 292 272 B1**
(45) Veröffentlichungstag und Bekanntmachung des Hinweises auf die Patenterteilung: **07.03.2007**
(21) Anmeldenummer: 01960318.2
(22) Anmeldetag: 15.06.2001
(51) Int. Cl.: A61K 8/41, A61Q 5/10

(54) **OXIDATIONSHAARFÄRBEMITTEL**
OXIDATION DYE FOR HAIR
COLORANT D'OXYDATION POUR LES CHEVEUX

(30) Priorität: 23.06.2000 DE 10030890
(43) Veröffentlichungstag der Anmeldung: 19.03.2003
(73) Patentinhaber: Henkel Kommanditgesellschaft auf Aktien, 40589 Düsseldorf (DE)
(72) Erfinder: ROSE, David, 40723 Hilden (DE)
(86) Internationale Anmeldenummer: PCT/EP2001/006809
(87) Internationale Veröffentlichungsnummer: WO 2001/097766

(56) Entgegenhaltungen:
- EP-A- 0 467 026
- DE-A- 4 028 661
- DE-A- 4 206 416
- DE-C- 19 730 412
- DE-C- 19 813 937
- US-A- 4 003 699

## Beschreibung

Diese Erfindung betrifft Oxidationsfärbemittel, die in einem wäßrigen Träger wenigstens ein 5-Halo-2,4-diaminoaIkylbenzoI als Kupplerkomponente sowie als Entwicklerkomponente wenigstens ein zweikerniges p-Phenylendiamin oder p-Aminophenol enthalten und deren Verwendung zum Färben von Keratinfasern, insbesondere menschlichen Haaren sowie ein entsprechendes Verfahren.

Für das Färben von Keratinfasern, insbesondere von Haaren, spielen die sogenannten Oxidationsfärbemittel wegen ihrer intensiven Farben und guten Echtheitseigenschaften, die bei relativ niedriger Färbetemperatur und in kurzen Färbezeiten erzielt werden, eine bevorzugte Rolle. Solche Färbemittel enthalten in einem geeigneten, meist wässrigen Träger eine Oxidationsbase, die auch als Entwicklerkomponente bezeichnet wird, und die unter dem Einfluß von Luftsauerstoff oder von Oxidationsmitteln durch oxidative Polymerisation einen Farbstoff ausbildet. Dieser Farbstoff kann durch Kupplung mit anderen Entwicklerverbindungen oder mit sogenannten Kupplerverbindungen, die selbst keine brauchbaren Farbstoffe ausbilden können, intensiviert und in der Nuance modifiziert werden.

Gute Oxidationsfarbstoffvorprodukte sollen in erster Linie folgende Voraussetzungen erfüllen: Sie müssen bei der oxidativen Kupplung die gewünschten Farbnuancen in ausreichender Intensität und Echtheit ausbilden. Sie müssen ferner ein gutes Aufziehvermögen auf die Faser besitzen, wobei insbesondere bei menschlichen Haaren keine merklichen Unterschiede zwischen strapaziertem und frisch nachgewachsenem Haar bestehen dürfen (Egalisiervermögen). Sie sollen beständig sein gegen Licht, Wärme, Reibung und den Einfluß chemischer Reduktionsmittel, z.B. Dauerwellenflüssigkeiten. Schließlich sollen sie - falls als Haarfärbemittel zur Anwendung kommend - die Kopfhaut nicht zu sehr anfärben, und vor allem sollen sie in toxikologischer und dermatologischer Hinsicht unbedenklich sein. Weiterhin soll die erzielte Färbung durch Blondierung leicht wieder aus dem Haar entfernt werden können, falls sie doch nicht den individuellen Wünschen der einzelnen Person entspricht und rückgängig gemacht werden soll.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2'-hydroxyethyl)-p-phenylendiamin, 2-(2',5'-Diaminophenyl)-ethanol, 2-(2',5'-Dianiinophenoxy)-ethanol, 1-Phenyl-3-boxyamido-4-amino-pyrazolon-5, 4-Amino-3-methylphenol, 2-Aminomethyl-4-aminophenol, 2-Hydroxy-4,5,6-triaminopyrimidin, 2,4-Dihydroxy-5,6-diaminopyrimidin, 2,5,6-Triamino-4-hydroxypyrimidin und 1,3-N,N'-Bis(2'-hydroxyethyl)-N,N'-bis(4'-aminophenyl)-diaminopropan-2-ol.

Als Kupplerkomponenten werden in der Regel m-Phenylendiaminderivate, Naphthole, Resorcin und Resorcinderivate, Pyrazolone und m-Aminophenole verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, 2-Chlor-resorcin, 4-Chlor-resorcin, 2-Methylresorcin, 5-Methylresorcin, m-Phenylendiamin, 2,4-Diaminophenoxyethanol, 1-Phenyl-3-methyl-pyrazolon-5, 2,4-Dichlor-3-aminophenol, 1,3-Bis-(2',4'-diaminophenoxy)-propan, 2-Chlor-6-methyl-3-aminophenol, 2-Methyl-4-chlor-5-aminophenol und 2-Amino-3-hydroxypyridin.

5-Halo-2,4-diaminoalkylbenzole sind als Kuppler für Oxidationsfärbemittel z.B. aus WO 92/04005 A1 bekannt. In dieser Druckschrift wird über das 5-Chlor-2,4-diaminotoluol lediglich ausgesagt, daß es mit p-Phenylendiamin blaue und mit p-Aminophenol schwache rötliche Nuancen liefert.

Es wurde nun aber gefunden, daß 5-Halo-2,4-diaminoalkylbenzole, in denen Halo für Fluor, Chlor oder Brom und Alkyl für eine C₁-C₄-Alkylgruppe steht, mit zweikernigen Entwicklerkomponenten besonders tiefe, licht- und waschechte Färbungen im Türkis- und Blaubereich liefern.

Gegenstand der Erfindung sind daher Oxidationsfärbemittel zum Färben von Keratinfasern, insbesondere von Haaren, die in einem wäßrigen Träger
(A) als Kupplerkomponente wenigstens ein 5-Halo-2,4-diamino-alkylbenzol und
(B) als Entwicklerkomponente mindestens eine zweikernige Entwicklerkomponente
enthalten.

Unter Keratinfasern sind dabei Wolle, Federn und Pelze, insbesondere aber menschliche Haare zu verstehen.

Unter zweikernigen Entwicklerkomponenten werden erfindungsgemäß Verbindungen verstanden, die mindestens zwei aromatische Kerne enthalten, die mindestens zwei gegebenenfalls substituierte Amino- und/oder Hydroxygruppen tragen, die in para- und/oder ortho-Stellung zueinander stehen.

Die Entwickler- und Kupplerkomponenten können auch in Form ihrer wasserlöslichen Säureadditionssalze, z.B. als Hydrochloride, Sulfate, Phosphate, Lactate, Acetate oder Glycolate enthalten sein.

Die 5-Halo-2,4-diaminoalkylbenzole können in der 5-Position mit Fluor, Chlor oder Brom substituiert sein und in der 1-Position eine C₁-C₄-Alkylgruppe tragen. In einer bevorzugten Ausführung der Erfindung ist als Kuppler das 5-Chlor-2,4-diaminotoluol enthalten.

Unter den zweikernigen Entwicklerkomponenten, die in den Färbezusammensetzungen gemäß der Erfindung verwendet werden können, kann man insbesondere die Verbindungen nennen, die der folgenden Formel (I) entsprechen, sowie ihre physiologisch verträglichen Salze: 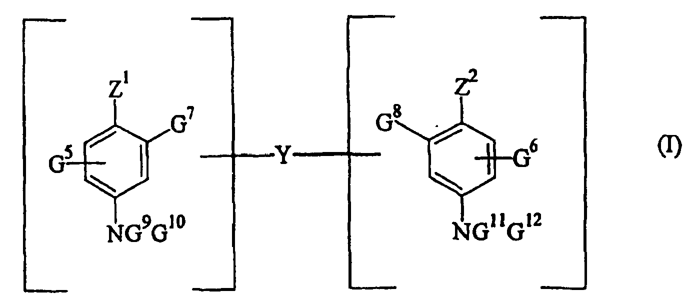 wobei:
- Z¹ und Z² stehen unabhängig voneinander für einen Hydroxyl- oder NH₂-Rest, der gegebenenfalls durch einen C₁- bis C₄-Alkylrest, durch einen C₁- bis C₄-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁- bis C₈-Alkoxyreste substituiert sein kann, oder eine direkte Bindung,
- G⁵ und G⁶ stehen unabhängig voneinander für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen C₁- bis C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁- bis C₄-Alkylrest, mit den Maßgaben, daß
- die Verbindungen der Formel (I) nur eine Verbrückung Y pro Molekül enthalten und
- die Verbindungen der Formel (I) mindestens eine Aminogruppe enthalten, die mindestens ein Wasserstoffatom trägt.

Beispiele für die als Substituenten im Rahmen dieser Anmeldung genannten C₁-C₄-Alkylreste sind die Gruppen Methyl, Ethyl, Propyl, Isopropyl, Butyl, Isobutyl und tert.-Butyt. Ethyl und Methyl sind bevorzugte Alkylreste. Beispiele für bevorzugte C₂-C₄-Alkenylreste sind Vinyl und Allyl. Erfindungsgemäß bevorzugte C₁-C₄-Alkoxyreste sind beispielsweise eine Methoxy- oder eine Ethoxygruppe. Weiterhin können als bevorzugte Beispiele für eine C₁-C₄-Monohydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Ein Beispiel für eine bevorzugte C₂-C₄-Polyhydroxyalkylgruppe ist die α,β-Dihydroxyethylgruppe. Beispiele für Halogenatome sind erfindungsgemäß F-, Cl-, Br oder I-Atome, Cl-Atome sind ganz besonders bevorzugt. Die weiteren verwendeten Begriffe leiten sich erfindungsgemäß von den hier gegebenen Definitionen ab.

Bevorzugte zweikernige Entwicklerkomponenten der Formel (I) sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, N,N-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (I) sind N,N'-Bis-(β-hydroxyethyl)-N,N'-bis-(4'-aminophenyl)-1,3-diamino-propan-2-ol, Bis-(2-hydroxy-5-aminophenyl)-methan, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Die zweikernigen Entwicklerverbindungen der Formel (I) sind bevorzugt symmetrische Verbindungen, d.h. beide Phenylreste weisen das gleiche Substitutionsmuster in Relation zur Verbrückung Y auf.

Als Beispiele für erfindungsgemäß geeignete zweikernige Entwickler der Formel (I) sind weiterhin zu nennen:
- Bis-(2-hydroxy-5-aminophenyl)-methan
- 1,3-Bis-(2-hydroxy-5-aminophenoxy)-propan
- 1,3-Bis-(2,5-diaminophenoxy)-propan
- Bis-(2,5-diaminophenyl)methan
- N,N'-Bis-(2-hydroxy-5-aminobenzyl)-piperazin
- N,N'-Bis-(2,5-diaminobenzyl)-piperazin
- N,N'-Bis-(2-hydroxyethyl)-N,N'-Bis-(4-aminophenyl)-1,3-diaminopropan-2-ol
- N,N'-Bis-(4-hydroxyphenyl)-ethylendiamin
- N,N'-Bis-(4-aminophenyl)-propylendiamin-1,3
- N,N'-Bis-(4-hydroxyphenyl)-piperazin
- N,N'-Bis-(4-hydroxyphenyl)-1,4-diazacycloheptan
- N,N'-Bis-(4-aminophenyl)-piperazin

Die Mittel können weiterhin ein Pyrimidinderivat enthalten.

Besonders bevorzugte Pyrimidinderivate sind ausgewählt aus der Gruppe 2,4,5,6-Tetraaminopyrimidin, 4-Hydroxy-2,5,6-triaminopyrimidin oder 2-Hydroxy-4,5,6-triaminopyrimidin.

Zur Nuancierung und Formulierung natürlich wirkender blonder, brauner oder schwarzer Haarfärbemittel können weitere Kuppler- und/oder Entwicklerverbindungen enthalten sein.

Besonders intensive blaue Nuancen können auch dadurch erzielt werden, daß man zwei oder mehr der erfindungsgemäß geeigneten Entwicklerkomponenten der Formeln I oder der Pyrimidine mit wenigstens einem erfindungsgemäßen Kuppler, bevorzugt mit 5-Chlor-2,4-diaminotoluol kombiniert.

Es kann erfindungsgemäß bevorzugt sein, als weitere Entwicklerkomponente ein p-Phenylendiaminderivat oder eines seiner physiologisch verträglichen Salze einzusetzen. Besonders bevorzugt sind p-Phenylendiaminderivate der Formel (E1) 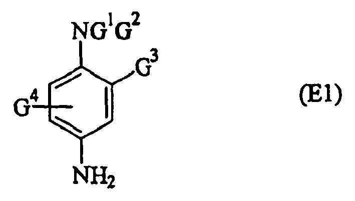 wobei
- G¹ steht für ein Wasserstoffatom, einen C₁-C₄-Alkylrest, einen C₁-C₄-Monohydroxyalkylrest, einen C₂-C₄-Polyhydroxyalkylrest, einen (C₁-C₄)-Alkoxy-(C₁-C₄)-alkylrest, einen 4'-Aminophenylrest oder einen C₁-C₄-Alkylrest, der mit einer stickstoffhaltigen Gruppe, einem Phenyl- oder einem 4'-Aminophenylrest substituiert ist,
- G² steht für ein Wasserstoffatom, einen C₁-C₄-Alkylrest, einen C₁-C₄-Monohydroxyalkylrest, einen C₂-C₄-Polyhydroxyalkylrest, einen (C₁-C₄)-Alkoxy-(C₁-C₄)-alkylrest oder einen C₁-C₄-Alkylrest, der mit einer stickstoffhaltigen Gruppe substituiert ist,
- G³ steht für ein Wasserstoffatom, ein Halogenatom, wie ein Chlor-, Brom-, Iod- oder Fluoratom, einen C₁-C₄-Alkylrest, einen C₁-C₄-Monohydroxyalkylrest, einen C₂-C₄-Potyhydroxyalkylrest, einen C₁-C₄-Hydroxyalkoxyrest, einen C₁-C₄-Acetylaminoalkoxyrest, einen C₁-C₄-Mesylaminoalkoxyrest oder einen C₁-C₄-Carbamoylaminoalkoxyrest,
- G⁴ steht für ein Wasserstoffatom, ein Halogenatom oder einen C₁-C₄-Alkylrest oder
- wenn G³ und G⁴ in ortho-Stellung zueinander stehen, können sie gemeinsam eine verbrückende α,ω-Alkylendioxogruppe, wie beispielsweise einen Ethylendioxygruppe bilden.

Beispiele für stickstoffhaltige Gruppen der Formel (E1) sind insbesondere die Aminogruppen, C₁-C₄-Monoalkylaminogruppen, C₁-C₄-Dialkylaminogruppen, C₁-C₄-Trialkylammoniumgruppen, C₁-C₄-Monohydroxyalkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroayethyl)amino-2-methylanilin, 4-N,N-Bis-(β-Hydroxyethyl)amino-2-chloranilin, 2-(β-Hydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Erfindungsgemäß ganz besonders bevorzugte p-Phenylendiaminderivate der Formel (E1) sind p-Phenylendiamin, p-Toluylendiamin, 2-(β-Hydroxyethyl)-p-phenylendiamin und N,N-Bis-(β-hydroxyethyl)-p-phenylendiamin.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E2) 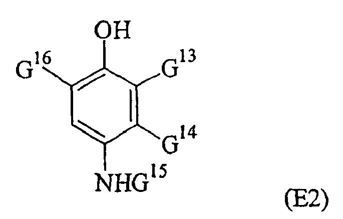 wobei
- G¹³ steht für ein Wasserstoffatom, ein Halogenatom, einen C₁-C₄-Alkylrest, einen C₁-C₄-Monohydroxyalkylrest, einen C₂-C₄-Polyhydroxyalkylrest, einen (C₁-C₄)-Alkoxy-(C₁-C₄)-alkylrest, einen C₁-C₄-Aminoalkylrest, einen Hydroxy-(C₁-C₄)-alkylaminorest, einen C₁-C₄-Hydroxyalkoxyrest, einen C₁-C₄-Hydroxyalkyl-(C₁-bis C₄)-aminoalkylrest oder einen (Di-C₁-C₄-Alkylamino)-(C₁-C₄)-alkylrest, und
- G¹⁴ steht für ein Wasserstoff- oder Halogenatom, einen C₁-C₄-Alkylrest, einen C₁-C₄-Hydroxyalkylrest, einen C₂-C₄-Polyhydroxyalkylrest, einen (C₁-C₄)-Alkoxy-(C₁-C₄)-alkylrest, einen C₁-C₄-Aminoalkylrest oder einen C₁- C₄-Cyanoalkylrest,
- G¹⁵ steht für Wasserstoff, einen C₁-C₄-Alkylrest, einen C₁-C₄-Hydroxyalkylrest, einen C₂-C₄-Polyhydroxyalkylrest, einen Phenylrest oder einen Benzylrest, und
- G¹⁶ steht für Wasserstoff oder ein Halogenatom.

Die in Formel (E2) verwendeten Substituenten sind erfindungsgemäß analog zu den obigen Ausführungen definiert.

Bevorzugte p-Aminophenole der Formel (E2) sind insbesondere p-Aminophenol, N-Methylp-Aminophenol, 4-Amino-3-methylphenol, 4-Amino-3-fluorphenol, 2-Hydroxymethylamino-4-aminophenol, 4-Amino-3-hydroxymethylphenol, 4-Amino-2-(2-hydroxyethoxy)phenol, 4-Amino-2-meihylphenol, 4-Amino-2-hydroaymethylphenol, 4-Amino-2-methoxymethylphenol, 4-Amino-2-aminomethylphenol, 4-Amino-2-(β-hydroxyethyl-aminomethyl)phenol, 4-Amino-2-fluorphenol, 4-Amino-2-chlorphenol, 2,6-Dichlor-4-aminophenol, 4-Amino-2-((diethylamino)methyl)phenol sowie ihre physiologisch verträglichen Salze.

Besonders bevorzugte Verbindungen der Formel (E2) sind p-Aminophenol, 4-Amino-3-methylphenol, 4-Amino-2-aminomethylphenol und 4-Amino-2-((diethylamino)methyl)phenol.

Ferner kann die Entwicklerkomponente ausgewählt sein aus o-Aminophenol und seinen Derivaten, wie beispielsweise 2-Amino-4-methylphenol oder 2-Amino-4-chlorphenol.

Weiterhin kann die Entwicklerkomponente ausgewählt sein aus heterocyclischen Entwicklerkomponenten, wie beispielsweise den Pyridin-, Pyrazol-, Pyrazol-PyrimidinDerivaten und ihren physiologisch verträglichen Salzen. Bevorzugt werden erfindungsgemäß Pyrazolderivate.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740931 und DE 195 43 988 beschrieben werden, wie 4,5-Diamino-1-methylpyrazol, 4,5-Diamino-1-(β-hydroxyethyl)-pyrazol, 3,4-Diaminopyrazol, 4,5-Diamino-1-(4'-chlorobenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2'-aminoethyl)amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4(β-hydroxyethyl)amino-1-methylpyrazol.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4-Methoxyphenyl)amino-3-amino-pyridin, 2,3-Diamino-6-methoxy-pyridin, 2-(β-Methoxyethyl)amino-3-amino-6-methoxy-pyridin und 3,4-Diamino-pyridin.

Bevorzugte Pyrazol-Pyrimidin-Derivate sind insbesondere die Derivate des Pyrazol-[1,5-a]-pyrimidin der folgenden Formel (E3) und dessen tautomeren Formen, sofern ein tautomeres Gleichgewicht besteht: 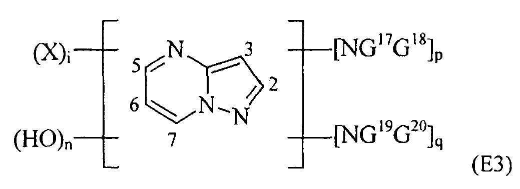 wobei:
- G¹⁷, G¹⁸, G¹⁹ und G²⁰ unabhängig voneinander stehen für ein Wasserstoffatom, einen C₁-C₄-Alkylrest, einen Aryl-Rest, einen C₁-C₄-Hydroxyalkylrest, einen C₂-C₄-Polyhydroxyalkylrest einen (C₁-C₄)-Alkoxy-(C₁-C₄)-alkylrest, einen C₁-C₄-Aminoalkylrest, der gegebenenfalls durch einen Acetyl-Ureid- oder Sulfonyl-Rest geschützt sein kann, einen (C₁-C₄)-Alkylamino-(C₁-C₄)-alkylrest, einen Di-[(C₁-C₄)-alkyl]-(C₁-C₄)-aminoalkylrest, wobei die Dialkyl-Reste gegebenenfalls einen Kohlenstoffzyklus oder einen Heterozyklus mit 5 oder 6 Kettengliedern bilden, einen C₁-C₄-Hydroxyalkyl- oder einen Di-(C₁-C₄)-[Hydroxyalkyl]-(C₁-C₄)-aminoalkylrest,
- die X-Reste stehen unabhängig voneinander für ein Wasserstoffatom, einen C₁-C₄-Alkylrest, einen Aryl-Rest, einen C₁-C₄-Hydroxyalkylrest, einen C₂-C₄-Polyhydroxyalkylrest, einen C₁-C₄-Aminoalkylrest, einen (C₁-C₄)-Alkylamino-(C₁-C₄)-alkylrest, einen Di-[(C₁-C₄)alkyl]- (C₁-C₄)-aminoalkylrest, wobei die Dialkyl-Reste gegebenenfalls einen Kohlenstoffzyklus oder einen Heterozyklus mit 5 oder 6 Kettengliedern bilden, einen C₁-C₄-Hydroxyalkyl- oder einen Di-(C₁-C₄-hydroxyalkyl)-aminoalkylrest, einen Aminorest, einen C₁-C₄-Alkyl- oder Di-(C₁-C₄-hydroxyalkyl)-aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
mit der Maßgabe, daß
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (E3) verwendeten Substituenten sind erfindungsgemäß analog zu den obigen Ausführungen definiert.

Wenn das Pyrazol-[1,5-a]-pyrimidin der obenstehenden Formel (E3) eine Hydroxygruppe an einer der Positionen 2, 5 oder 7 des Ringsystems enthält, besteht ein tautomeres Gleichgewicht, das zum Beispiel im folgenden Schema dargestellt wird:

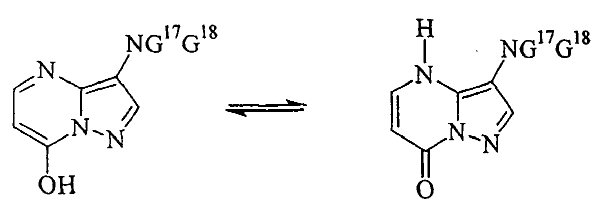

Unter den Pyrazol-[1,5-a]-pyrimidinen der obenstehenden Formel (E3) kann man insbesondere nennen:
- Pyrazol-[1,5-a]-pyrimidin-3,7-diamin;
- 2,5-Dimethyl pyrazol-[1,5-a]-pyrimidin-3,7-diamin;
- Pyrazol-(1,5-a]-pyrimidin-3,5-diamin;
- 2,7-Dimethyl pyrazol-[1,5-a]-pyrimidin-3,5-diamin;
- 3-Amino pyrazol-[1,5-a]-pyrimidin-7-ol;
- 3-Amino pyrazol-[1,5-a]-pyrimidin-5-ol;
- 2-(3-Amino pyrazol-[1,5-a]-pyrimidin-7-ylamino)-ethanol;
- 2-(7-Amino pyrazol-[1,5-a]-pyrimidin-3-ylamino)-ethanol;
- 2-[(3-Amino pyrazol-[1,5-a]-pyrimidin-7-yl)-(2-hydroxy-ethyl)-amino]-ethanol;
- 2-[(7-Amino pyrazol-[1,5-a]-pyrimidin-3-yl)-(2-hydroxy-ethyl)-amino]-ethanol;
- 5,6-Dimethyl pyrazol-[1,5-a]-pyrimidin-3,7-diamin;
- 2,6-Dimethyl pyrazol-[1,5-a]-pyrimidin-3,7-diamin;
- 2,5,N7,N7-Tetramethyl pyrazol-[1,5-a]-pyrimidin-3,7-diamin;
sowie ihre physiologisch verträglichen Salze und ihre tautomeren Formen, wenn ein tautomeres Gleichgewicht vorhanden ist.

Die Pyrazol-[1,5-a]-pyrimidine der obenstehenden Formel (E3) können wie in der Literatur beschrieben durch Zyklisierung ausgehend von einem Aminopyrazol oder von Hydrazin hergestellt werden.

Ganz besonders bevorzugte zusätzliche Entwicklerkomponenten sind ausgewählt aus p-Phenylendiamin, p-Toluylendiamin, 2-(β-Hydroxyethyl)-p-phenylendiamin, N,N-Bis-(β-hydroxyethyl)-p-phenylendiamin, 2-(2',5'-Diaminophenoxy)-ethanol, 4,4'-Diaminodiphenylamin, p-Aminophenol, o-Aminophenol, 4-Amino-3-methylphenol, 4-Amino-2-aminomethylphenol, 4-Amino-2-((diethylamino)methyl)phenol, 2-Hydroxyethylaminomethyl-4-amino-phenol, 4-Amino-3-fluorphenol, 2-Hydroxymethyl-4-aminophenol, 4-Amino-2-(β-hydroxyethoxy)-phenol, 2-Dimethylamino-4,5,6-triaminopyrimidin, 2,4-Dihydroxy-5,6-diaminopyrimidin, 2,5,6-Triaminopyrimidin, 1-Phenyl-3-carboxyamido-4-amino-pyrazol-5-on, 4,5-Diamino-1-methylpyrazol, 4,5-Diamino-1-(β-hydroxyethyl)-pyrazol, 3,4-Diaminopyrazol, 4,5-Diamino-1-(4'-chlorobenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxwyphenyl)-pyrazol, 4,5-Diamino-1 -ethyl-3-hydroxymethylpyrazol, 4,5-Diarnino-3-hydroxymethyl-1-methylpyrazol, 4,5-Diamino-3-hydroxymethyl-1-isopropylpyrazol, 4,5-Diamino-3-methyl-l-isopropylpyrazol, 4-Amino-5-(2'-aminoethyl)amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4(β-hydroxyethyl)amino-1-methylpyrazol.

In einer speziellen Ausführungsform werden zusätzliche Entwicklerkomponenten ausgewählt aus p-Phenylendiamin, p-Toluylendiamin, p-Aminophenol, o-Aminophenol, 1-(2'-Hydroxyethyl)-2,5-diaminobenzol, N,N-Bis-(2'-hydroxy-ethyl)-p-phenylendiamin, 2-(2',5'-Diaminophenoxy)-ethanol, 1-Phenyl-3-carboxyamido-4-amino-pyrazolon-5, 4-Amino-3-methylphenol, 2,4-Dihydroxy-5,6-diaminopyrimidin, 2-Dimethylamino-4,5,6-triaminopyrimidin, 2-Hydroxy-ethylaminomethyl-4-amino-phenol, 4,4'-Diaminodiphenylamin, 4-Amino-3-fluorphenol, 2-Aminomethyl-4-amino-phenol, 2-Hydroxymethyl-4-aminophenol, 4-Amino-2-(2'-hydroxyethoxy)-phenol sowie 4,5-Diaminopyrazol-Derivate nach EP 0 740 931 bzw. WO 94/08970 wie z.B. 4,5-Diamino-1-(2'-hydroxyethyl)-pyrazol.

Erfindungsgemäß bevorzugte weitere Kupplerkomponenten sind
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoracetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2',4'-diaminophenoxy)propan, 1-Methoxy-2-amino-4-(2'-hydroxyethylamino)-benzol, 1,3-Bis-(2',4'-diaminophenyl)propan, 2,6-Bis-(2'-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol.

In einer speziellen Ausführungsform sind erfindungsgemäß bevorzugte weitere Kupplerkomponenten ausgewählt aus 1-Naphthol, Pyrogallol, 1,5-, 2,7- und 1,7-Dihydroxynaphthalin, o-Aminophenol, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 4-Chlorresorcin, 2-Chlor-6-methyl-3-aminophenol, 2-Methylresorcin, 5-Methylresorcin, 2,5-Dimethylresorcin, 2,6-Dihydroxypyridin, 2,6-Diaminopyridin, 2-Amino-3-hydroxypyridin, 2,6-Dihydroxy-3,4-diaminopyridin, 3-Amino-2-methylamino-6-methoxypyridin, 2,6-Bis-(2'-hydroxyethylamino)-toluol, 2,4-Diaminophenoxyethanol, 1-Methoxy-2-amino-4-(2'-hydroxyethylamino)-benzol, 2-Methyl-4-chlor-5-amino-phenol, 6-Methyl-1,2,3,4-tetrahydrochinoxalin, 3,4-Methylendioxyphenol, 3,4-Methylendioxyanilin, 2,6-Dimethyl-3-aminophenol, 3-Amino-6-methoxy-2-methylaminophenol, 2-Hydroxy-4-aminophenoxyethanol, 2-Methyl-5-(2'-hydroxyethylamino)-phenol und 2,6-Dihydroxy-3,4-dimethylpyridin.

Die erfindungsgemäßen Haarfärbemittel enthalten sowohl die Entwicklerkomponenten als auch die Kupplerkomponenten bevorzugt in einer Menge von 0,005 bis 20 Gew.-%, vorzugsweise 0,1 bis 5 Gew.-%, jeweils bezogen auf das gesamte Oxidationsfärbemittel. Dabei werden Entwicklerkomponenten und Kupplerkomponenten im allgemeinen in etwa molaren Mengen zueinander eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten in einem Mol-Verhältnis von 1:0,5 bis 1:3, insbesondere 1:1 1 bis 1:2, enthalten sein können.

In einer weiteren Ausführungsform können zusätzlich Indole und/oder Indoline in den erfindungsgemäßen Haarfärbemitteln eingesetzt werden. Bevorzugt sind Indole und/oder Indoline, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe.

Besonders gut als Vorstufen naturanaloger Haarfarbstoffe geeignet sind Derivate des 5,6-Dihydroxyindolins der Formel (IIa), 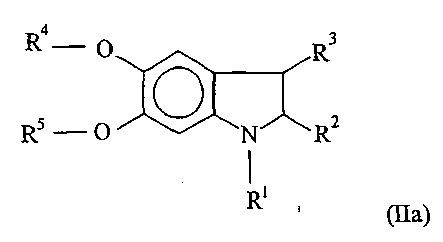 in der unabhängig voneinander
R¹ steht für Wasserstoff, eine C₁-C₄-Alkylgruppe, eine C₃-C₆-Cycloalkylgruppe, eine C₂-C₄-Alkenylgruppe oder eine C₁-C₄-Hydroxyalkylgruppe,
R² steht für Wasserstoff oder eine -COOH-Gruppe, wobei die -COOH-Gruppe auch als Salz mit einem physiologisch verträglichen Kation vorliegen kann,
R³ steht für Wasserstoff oder eine C₁-C₄-Alkylgruppe,
R⁴ steht für Wasserstoff, eine C₁-C₄-Alkylgruppe oder eine Gruppe -CO-R⁶, in der
R⁶ steht für eine C₁-C₄-Alkylgruppe, und
R⁵ steht für eine der unter R⁴ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (IIb), 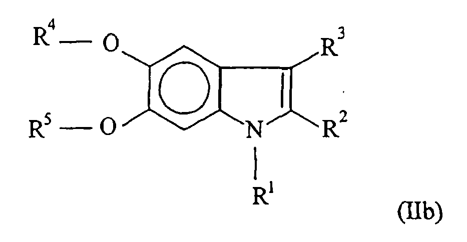 in der unabhängig voneinander
R¹ steht für Wasserstoff, eine C₁-C₄-Alkylgruppe, eine C₃-C₆-Cycloalkylgruppe, eine C₂-C₄-Alkenylgruppe oder eine C₁-C₄-Hydroxyalkylgruppe,
R² steht für Wasserstoff oder eine -COOH-Gruppe, wobei die -COOH-Gruppe auch als Salz mit einem physiologisch verträglichen Kation vorliegen kann,
R³ steht für Wasserstoff oder eine C₁-C₄-Alkylgruppe,
R⁴ steht für Wasserstoff, eine C₁-C₄-Alkylgruppe oder eine Gruppe -CO-R⁶, in der
R⁶ steht für eine C₁-C₄-Alkylgruppe, und
R⁵ steht für eine der unter R⁴ genannten Gruppen,
sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den erfindungsgemäßen Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-% enthalten.

Darüber hinaus können zur Erzielung bestimmter Färbewirkungen auch direktziehende Farbstoffe verwendet werden.

Bevorzugt enthalten die erfmdungsgemäßen Haarfärbemittel zur weiteren Modifizierung der Farbnuancen neben den Oxidationsfarbstoffvorprodukten zusätzlich übliche direktziehende Farbstoffe. Direktziehende Farbstoffe sind bevorzugt Nitrophenylendiamine, Nitroaminophenole, Azofarbstoffe, Anthrachinone oder Indophenole. Bevorzugte direktziehende Farbstoffe sind unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 12, Disperse Blue 3, HC Violet 1, Disperse Violet 1, Disperse Violet 4, Acid Violet 43, Disperse Black 9 und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol bekannt.

Ferner können die erfindungsgemäßen Mittel einen kationischen direktziehenden Farbstoff enthalten. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

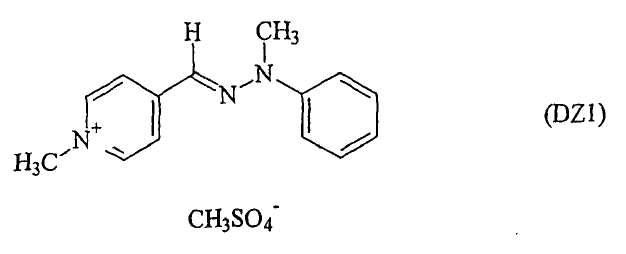

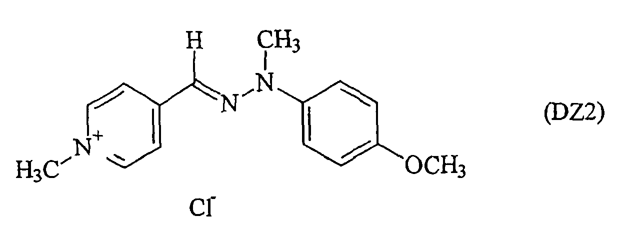

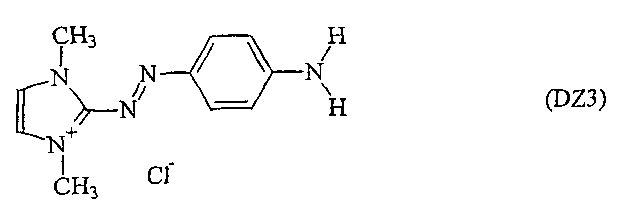

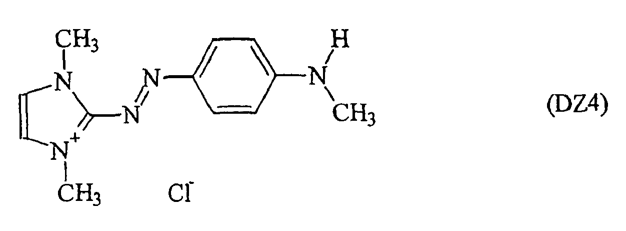

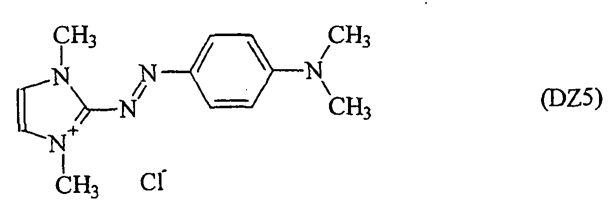

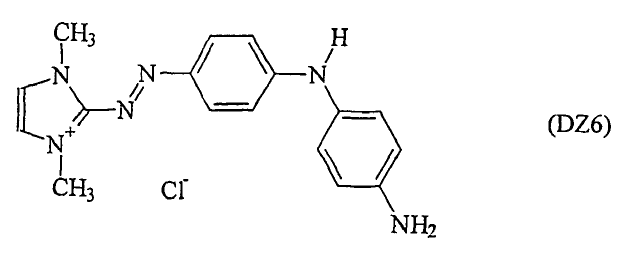

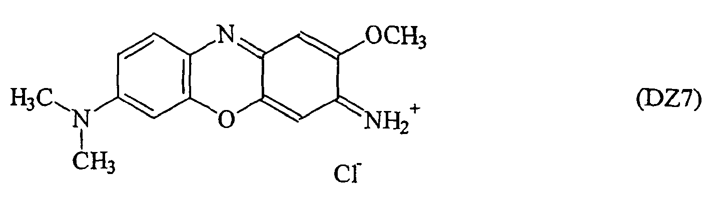

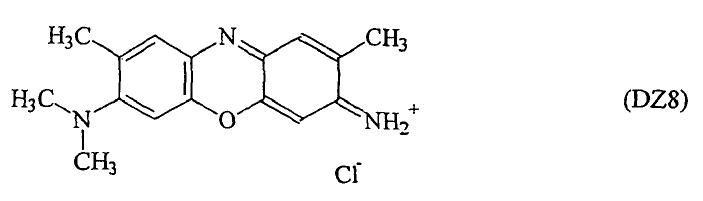

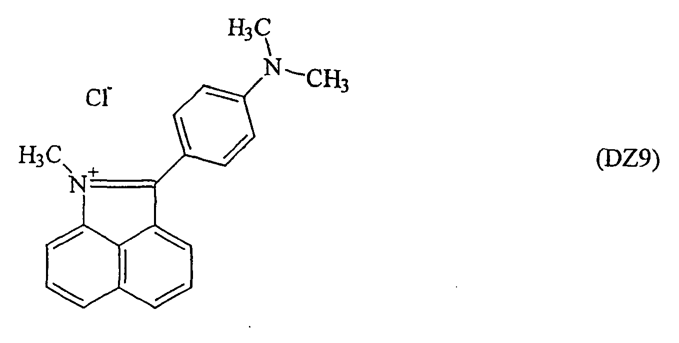

Die Verbindungen der Formeln (DZ1), (DZ3) und (DZ5) sind ganz besonders bevorzugte kationische direktziehende Farbstoffe der Gruppe (c). Die kationischen direktziehenden Farbstoffe, die unter dem Warenzeichen Arianor^{®} vertrieben werden, sind erfindungsgemäß besonders bevorzugte direktziehende Farbstoffe.

Besonders bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1, Disperse Violet 4, Disperse Black 9, Basic Brown 16 und Basic Brown 17 bekannten Verbindungen sowie 4-Amino-2-nitrodiphenylamin-2'-carbonsäure, 6-Nitro-1,2,3,4-tetrahydrochinoxalin, Hydroxyethyl-2-nitro-toluidin, Pikraminsäure, 2-Amino-6-chloro-4-nitrophenol 4-Ethylamino-3-nitrobenzoesäure und 2-Chloro-6-ethylamino-1-hydroxy-4-nitrobenzol.

Die erfindungsgemäßen Mittel gemäß dieser Ausführungsformen enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe, wie sie beispielsweise in Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten sind, enthalten.

Es ist nicht erforderlich, daß die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe), sowie Kapitel 8, Seiten 264-267; Oxidationsfarbstoffvorprodukte), erschienen als Band 7 der Reihe "Dermatology" (Hrsg.: Ch. Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Zur Herstellung der erfindungsgemäßen Oxidationsfärbemittel werden die Oxidationsfarbstoffvorprodukte in einen geeigneten wäßrigen Träger eingearbeitet. Solche Träger sind z.B. verdickte wäßrige Lösungen, Cremes (Emulsionen), Gele oder tensidhaltige schäumende Zubereitungen, z.B. Shampoos oder Schaumaerosole oder andere Zubereitungen, die für die Anwendung auf dem Haar geeignet sind.

Als Trägerkomponenten werden bevorzugt
- Netz- und Emulgiermittel
- Verdickungsmittel
- Reduktionsmittel (Antioxidantien)
- haarpflegende Zusätze
- Duftstoffe und
- Lösungsmittel wie z. B. Wasser, Glycole oder niedere Alkohole
eingesetzt.

Als Netz- und Emulgiermittel eignen sich z. B. anionische, zwitterionische, ampholytische und nichtionische Tenside. Auch kationische Tenside können zur Erzielung bestimmter Effekte eingesetzt werden.

In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z.B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)x-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Nichtionogene Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel R'O-(Z)ₓ. Diese Verbindungen sind durch die folgenden Parameter gekennzeichnet.

Der Alkylrest R' enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R¹ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R
- im wesentlichen aus C₈- und C₁₀-Alkylgruppen,
- im wesentlichen aus C₁₂- und C₁₄-Alkylgruppen,
- im wesentlichen aus C₈-C₁₆-Alkylgruppen oder
- im wesentlichen aus C₁₂-C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, daß eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z.B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Coming; ein stabilisiertes Trimethylsilylamodimethicon), Dow Coming 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquatemäre Polydimethylsiloxane, Quaternium-80).

Ein Beispiel für ein als kationisches Tensid einsetzbares quaternäres Zuckerderivat stellt das Handelsprodukt Glucquat^{®}100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Als Verdickungsmittel eignen sich einmal die wasserlöslichen hochmolekularen Polysaccharid-Derivate oder Polypeptide, z. B. Cellulose- oder Stärkeether, Gelatine, Pflanzengumme, Biopolymere (Xanthan-Gum) oder wasserlösliche synthetische Polymere wie z. B. Polyvinylpyrrolidon, Polyvinylalkohol, Polyethylenoxide, Polyacrylamide, Polyurethane, Polyacrylate und andere. Zum anderen kann man tensidhaltige Zubereitungen auch durch Solubilisierung oder Emulgierung von polaren Lipiden verdicken. Solche Lipide sind z. B. Fettalkohole mit 12 - 18 C-Atomen, (freie) Fettsäuren mit 12 - 18 C-Atomen, Fettsäurepartialglyceride, Sorbitanfettsäureester, Fettsäurealkanolamide, niedrig oxethylierte Fettsäuren oder Fettalkohole, Lecithine, Sterine. Schließlich kann man gelförmige Träger auch auf Basis wässriger Seifengele, z. B. von Ammonium-Oleat, erzeugen. Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol werden bevorzugt verwendet,

Reduktionsmittel (Antioxidantien), die dem Träger zugesetzt werden, um eine vorzeitige oxidative Entwicklung des Farbstoffs vor der Anwendung auf dem Haar zu verhindern, sind z. B. Natriumsulfit oder Natriumascorbat.

Haarpflegende Zusätze können z. B. Fette, Öle oder Wachse in emulgierter Form, strukturgebende Additive wie z. B. Glucose oder Pyridoxin, avivierende Komponenten wie z. B. wasserlösliche Proteine, Proteinabbauprodukte, Aminosäuren, wasserlösliche kationische Polymere, Silicone, Vitamine wie z.B. Panthenol oder Pflanzenextrakte sein.

Schließlich können Duftstoffe und Lösungsmittel wie z. B. Glycole wie 1,2-Propylenglycole, Glycerin, Glycolether wie z. B. Butylglycol, Ethyldiglycol oder niedere einwertige Alkohole wie Ethanol oder Isopropanol enthalten sein.

Zusätzlich können noch weitere Hilfsmittel enthalten sein, die die Stabilität und Anwendungseigenschaften der Oxidationsfärbemittel verbessern, z. B. Komplexbildner wie EDTA, NTA oder Organophosphonate, Quell- und Penetrationsmittel wie z. B. Harnstoff, Guanidin, Hydrogencarbonate, Puffersalze wie z. B. Ammoniumchlorid, Ammoniumcitrat, Ammoniumsulfat oder Alkanolammoniumsalze und gegebenenfalls Treibgase.

Weiterhin können die erfindungsgemäßen Mittel bevorzugt noch einen konditionierenden Wirkstoff, ausgewählt aus der Gruppe, die von kationischen Tensiden, kationischen Polymeren, Alkylamidoaminen, Paraffinölen, pflanzlichen Ölen und synthetischen Ölen gebildet wird, enthalten.

Als konditionierende Wirkstoffe bevorzugt sein können kationische Polymere. Dies sind in der Regel Polymere, die ein quartäres Stickstoffatom, beispielsweise in Form einer Ammoniumgruppe, enthalten.
Bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR°400 sind bevorzugte quaternierte Cellulose-Derivate.
- polymere Dimethyldiallylammoniumsalze und deren Copolymere mit Acrylsäure sowie Estern und Amiden von Acrylsäure und Methacrylsäure. Die unter den Bezeichnungen Merquat^{®} 100 (Poly(dimethyldiallylammoniumchlorid)), Merquat^{®}550 (Dimethyldiallylammoniumchlorid-Acrylamid-Copolymer) und Merquat^{®} 280 (Dimethyldiallylammoniumchlorid-Acrylsäure-Copolymer im Handel erhältlichen Produkte sind Beispiele für solche kationischen Polymere.
- Copolymere des Vinylpyrrolidons mit quaternierten Derivaten des Dialkylaminoacrylats und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat^{®}734 und Gafquat^{®}755 im Handel erhältlich.
- Vinylpyrrolidon-Methoimidazoliniumchlorid-Copolymere, wie sie unter der Bezeichnung Luviquat^{®} angeboten werden.
- quatemierter Polyvinylalkohol
sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Besonders bevorzugt sind kationische Polymere der vier erstgenannten Gruppen, ganz besonders bevorzugt sind Polyquaternium-2, Polyquaternium-10 und Polyquatemium-22.

Als konditionierende Wirkstoffe weiterhin geeignet sind Silikonöle, insbesondere Dialkyl- und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200, DC 344, DC 345 und DC 1401 vertriebenen Produkte sowie die Handelsprodukte Q2-7224 (Hersteller: Dow Corning; ein stabilisiertes Trimethylsilylamodimethicon), Dow Corning^{®} 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquatemäre Polydimethylsiloxane, Quaternium-80).

Ebenfalls einsetzbar als konditionierende Wirkstoffe sind Paraffinöle, synthetisch hergestellte oligomere Alkene sowie pflanzliche Öle wie Jojobaöl, Sonnenblumenöl, Orangenöl, Mandelöl, Weizenkeimöl und Pfirsichkernöl.

Gleichfalls geeignete haarkonditionierende Verbindungen sind Phospholipide, beispielsweise Sojalecithin, Ei-Lecithin und Kephaline.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vemetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butyl-acrylamid-Terpolymere,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessemde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoacarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, N₂O, Dimethylether, CO₂ und Luft,
- Antioxidantien.

Bezüglich weiterer fakultativer Komponenten sowie den eingesetzten Mengen dieser Komponenten wird ausdrücklich auf die dem Fachmann bekannten einschlägigen Handbücher, z.B. K. Schrader, Grundlagen und Rezepturen der Kosmetika, 2. Auflage, Hüthig Buch Verlag, Heidelberg, 1989, verwiesen.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen Persulfate, Chlorite und insbesondere Wasserstoffperoxid oder dessen Anlagerungsprodukte an Harnstoff, Melamin sowie Natriumborat in Frage. Man kann aber das Oxidationsfärbemittel auch zusammen mit einem Katalysator auf das Haar aufbringen, der die Oxidation der Farbstoffvorprodukte, z.B. durch Luftsauerstoff, aktiviert. Solche Katalysatoren sind z.B. Übergangsmetallverbindungen, Iodide, Chinone oder bestimmte Enzyme. Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.
- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Ein weiterer Gegenstand der Erfindung ist daher ein Verfahren zum Färben von Keratinfasern, insbesondere von Haaren, bei dem man auf die Faser ein erfindungsgemäßes Oxidationsfärbemittel zusammen mit einem Oxidationsmittel und/oder mit einem Katalysator zur Aktivierung der Oxidation aufbringt und nach einer Einwirkungszeit wieder mit Wasser oder mit einer wäßrigen Tensidzubereitung abspült.

Zweckmäßigerweise wird die Zubereitung des Oxidationsmittels unmittelbar vor dem Haarefärben mit der Zubereitung aus den Oxidationsfarbstoffvorprodukten vermischt. Das dabei entstehende gebrauchsfertige Haarfärbepräparat sollte bevorzugt einen pH-Wert im Bereich von 6 bis 10 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von ca. 30 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Ein dritter Gegenstand der Erfindung ist die Verwendung der erfindungsgemäßen Oxidationshaarfärbemittel zum Färben von Keratinfasern, insbesondere menschlichen Haaren.

Die nachfolgenden Beispiele sollen den Erfindungsgegenstand näher erläutern.

### Beispiele

Es wurde eine Basis-Creme der folgenden Zusammensetzung hergestellt:

| | |
|---|---|
| Hydrenol^{®}D¹ | 17,00 g |
| Lorol^{®}techn.² | 4,00 g |
| Eumulgin^{®}B2³ | 1,50 g |
| Texapon^{®}NSO⁴ | 40,00 g |
| Dehyton^{®}K⁵ | 25,00 g |
| Wasser | 12,50 g |

| | |
|---|---|
| ¹ C₁₆-C₁₈-Fettalkohol (INCI-Bezeichnung: Cetearyl alcohol) (COGNIS) ² C₁₂-C₁₈-Fettalkohol (INCI-Bezeichnung: Coconut alcohol) (COGNIS) ³ Cetearylstearylalkohol mit ca. 20 EO-Einheiten (INCI-Bezeichnung: Ceteareth-20) (COGNIS) ⁴ Laurylethersulfat, Natriumsalz (ca. 27,5 % Aktivsubstanz; (INCI-Bezeichnung: Sodium Laureth Sulfate) (COGNIS) ⁵ N,N-Dimethyl-N-(C₈-C₁₈-kokosamidopropyl)ammoniumacetobetain (ca. 30 % Aktivsubstanz; INCI-Bezeichnung: Aqua (Water), Cocamidopropyl Betaine) (COGNIS) | |

Die Substanzen Hydrenol^{®}D, Lorol^{®} und Eumulgin^{®}B2 wurden bei 80°C aufgeschmolzen, mit dem 80°C heißem Wasser, enthaltend Texapon^{®}NSO und Dehyton^{®}K, vermischt und unter starkem Rühren emulgiert. Danach wurde die Emulsion unter schwachem Rühren abgekühlt.

Es wurden Färbecremes der folgenden Zusammensetzung hergestellt:

| | |
|---|---|
| Basiscreme | 50,0 g |
| Entwicklerkomponente | 7,5 mmol |
| 5-Chlor-2,4-diaminotoluol | 1,173 g (7,5 mmol) |
| Na₂SO₃ (Inhibitor) | 1,0 g |
| (NH₄)₂SO₄ | 1,0 g |
| Konz. NH₃-Lösung | ad pH= 10 |
| Wasser | ad 100 g |

Für die Ausfärbung wurden folgende Entwicklerverbindungen verwendet:
E1: Bis-(2-hydroxy-5-aminophenyl)-methan
E2: N,N'-Bis-(2-hydroxyethyl)-N,N'-Bis-(4-aminophenyl)-1,3-diaminopropan-2-ol.
E3: N,N'-Bis-(4-aminophenyl)-1,4-diazacycloheptan
E4: 2,4,5,6-Tetraaminopyrimidin
E5: 2,5-Diaminophenylethanol
E6: 2-Aminomethyl-4-aminophenol

Es wurden Kombinationen von je 3,75 mmol der einzelnen Entwickler eingesetzt, so daß die Gesamtmenge an Entwicklerkomponente 7,5 mmol pro 100 g betrug.

Die Bestandteile wurden der Reihe nach miteinander vermischt. Nach Zugabe der Oxidationsfarbstoffvorprodukte, des Inhibitors und des (NH₄)₂SO₄ wurde mit konzentrierter Ammoniaklösung der pH-Wert der Emulsion auf 10 eingestellt, dann wurde mit Wasser auf 100 g aufgefüllt.

Die Entwicklung der Färbung wurde in einer Versuchsreihe mit Luftsauerstoff, in einer zweiten Versuchsreihe mit 1 Gew.-%-iger Wasserstoffperoxidlösung und in einer dritten Versuchsreihe mit 9 Gew.-%-iger Wasserstoffperoxid-Lösung durchgeführt.

Hierzu wurden zu 100 g der Emulsion
- in der ersten Versuchsreihe 50 g Wasser
- in der zweiten Versuchsreihe 50 g H₂O₂-Lösung (1%-ig), und
- in der dritten Versuchsreihe 50 g H₂O₂-Lösung (9%-ig)
zugemischt.

Die gebrauchsfertigen Färbeansätze wurden dann auf 5 cm lange Strähnen standardisierten, zu 80 % ergrauten, aber nicht besonders vorbehandelten Menschenhaars (Fa. Kerling) aufgetragen. Nach 30 Minuten Einwirkzeit bei 32°C wurde das Haar mit Wasser gespült, mit einem üblichen Shampoo ausgewaschen, erneut gespült und getrocknet.

Die Ergebnisse der Färbeversuche sind der Tabelle zu entnehmen:

| Beispiel | Entwickler | Luftoxidation | 1 Gew.-% H₂O₂ | 9 Gew.-% H₂O₂ |
|---|---|---|---|---|
| 1 | E1 + E2 | mattgrün | graublau | tiefblau |
| 2 | E1 + E3 | grautürkis | dunkelblau | dunkelblau |
| 3 | E2 + E3 | grautürkis | dunkelblau | dunkelblau |
| 4 | E3 + E4 | tiefgrün | dunkeltürkis | dunkelblau |
| 5 | E2 + E5 | grautürkis | dunkelblau | lapisblau |
| 6 | E1 + E6 | nougatfarbig | haarbraun | graubraun |

## Patentansprüche

1. Oxidationsfärbemittel zum Färben von Keratinfasern, insbesondere von Haaren, die in einem wässrigen Träger
(A) als Kupplerkomponente wenigstens ein 5-Halo-2,4-diamino-alkylbenzol und
(B) als Entwicklerkomponenten mindestens eine zweikernige Entwicklerkomponente
enthalten.

2. Oxidationsfärbemittel gemäß Anspruch 1, **dadurch gekennzeichnet, dass** das als Kuppler fungierende 5-Halo-2,4-diamino-alkylbenzol das 5-Chlor-2,4-diaminotoluol ist.

3. Mittel nach einem der Ansprüche 1 oder 2, **dadurch gekennzeichnet, dass** die zweikemige Entwicklerkomponenten eine Verbindung der Formel (I) ist 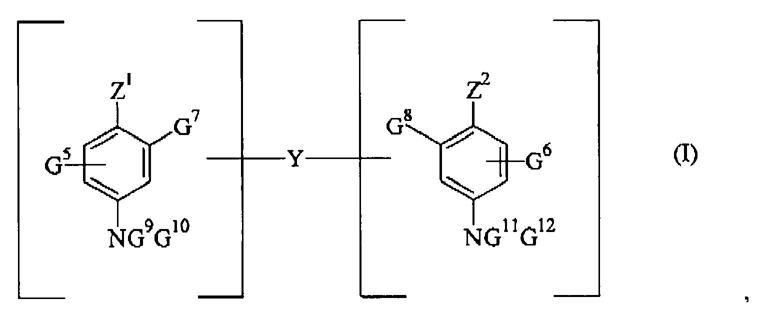 wobei:
- Z¹ und Z² stehen unabhängig voneinander für einen Hydroxyl- oder NH₂-Rest, der gegebenenfalls durch einen C₁- bis C₄-Alkylrest, durch einen C₁- bis C₄-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁- bis C₈-Alkoxyreste substituiert sein kann, oder eine direkte Bindung,
- G⁵ und G⁶ stehen unabhängig voneinander für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen C₁- bis C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁- bis C₄-Alkylrest,
mit den Maßgaben, dass
- die Verbindungen der Formel (I) nur eine Verbrückung Y pro Molekül enthalten und
- die Verbindungen der Formel (I) mindestens eine Aminogruppe enthalten, die mindestens ein Wasserstoffatom trägt.

4. Mittel nach Anspruch 3, **dadurch gekennzeichnet, dass** die Verbindung der Formel (I) ausgewählt ist aus N,N'-Bis-(β-hydroxyethyl)-N,N'-bis-(4'-aminophenyl)-1,3-diaminopropan-2-ol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihren physiologisch verträglichen Salzen.

5. Mittel nach Anspruch 4, **dadurch gekennzeichnet, dass** die Verbindung der Formel (I) ausgewählt ist aus N,N'-Bis-(β-hydroxyethyl)-N,N'-bis-(4'-aminophenyl)-1,3-diaminopropan-2-ol, Bis-(2-hydroxy-5-aminophenyl)-methan, N,N'-Bis-(4'-aminophenyl)-1,4, diazacycloheptan und 1,10-Bis-(2',5'-diaminophenyl)-1,4,7,10-tetraoxadecan und ihren physiologisch verträglichen Salzen.

6. Mittel nach einem der Ansprüche 1 bis 5, **dadurch gekennzeichnet, dass** es weiterhin mindestens ein Pyrimidinderivat enthält.

7. Mittel nach Anspruch 6, **dadurch gekennzeichnet, dass** das Pyrimidinderivat ausgewählt ist aus der Gruppe 2,4,5,6-Tetraaminopyrimidin, 4-Hydroxy-2,5,6-triazminopyrimidin und 2-Hydroxy-4,5,6-triaminopyrimidin sowie deren physiologisch verträglichen Salzen.

8. Mittel nach einem der Ansprüche 1 bis 7, **dadurch gekennzeichnet, dass** es mindestens eine weiteres Oxidationsfarbstoffvorprodukt vom Entwickler- oder Kupplertyp enthält.

9. Mittel nach einem der Ansprüche 1 bis 8, **dadurch gekennzeichnet, dass** es weiterhin mindestens ein Farbstoffvorprodukt vom Indol- oder Indolintyp enthält.

10. Mittel nach einem der Ansprüche 1 bis 9, **dadurch gekennzeichnet, dass** es weiterhin mindestens einen direktziehenden Farbstoff enthält.

11. Verfahren zur Färbung von Keratinfasern, insbesondere von Haaren, **dadurch gekennzeichnet, dass** man auf die Fasern ein Oxidationsfärbemittel gemäß einem der Ansprüche 1 bis 10 zusammen mit einem Oxidationsmittel und/oder mit einem Katalysator zur Aktivierung der Oxidation aufbringt und nach einer Einwirkungszeit wieder mit Wasser oder einer wässrigen Tensidzubereitung abspült.

12. Verwendung von Oxidationsfärbemitteln gemäß einem der Ansprüche 1 bis 10 zum Färben von Keratinfasern, insbesondere menschlichen Haaren.

## Claims

1. Oxidation dyes for dyeing keratin fibres, in particular hair, which comprise, in an aqueous carrier,
(A) as coupler component at least one 5-halo-2,4-diaminoalkylbenzene and
(B) as developer component at least one binuclear developer component.

2. Oxidation dyes according to Claim 1, **characterized in that** the 5-halo-2,4-diaminoalkylbenzene acting as coupler is 5-chloro-2,4-diaminotoluene.

3. Agent according to one of Claims 1 or 2, **characterized in that** the binuclear developer component is a compound of the formula (I) 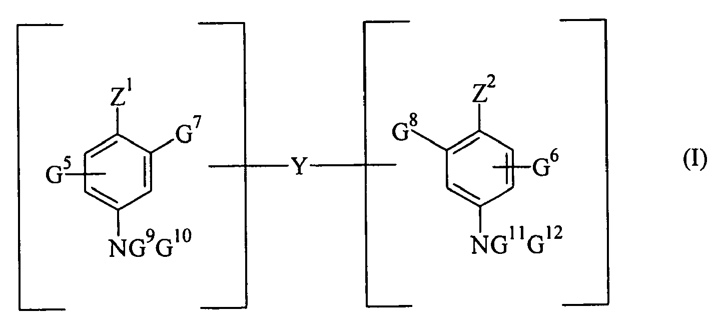 where:
- Z¹ and Z², independently of one another, are a hydroxyl or NH₂ radical, which is optionally substituted by a C₁- to C₄-alkyl radical, by a C₁- to C₄-hydroxyalkyl radical and/or by a bridge Y, or which is optionally part of a bridging ring system,
- the bridge Y is an alkylene group having 1 to 14 carbon atoms, such as, for example, a linear or branched alkylene chain or an alkylene ring, which may be interrupted or terminated by one or more nitrogen-containing groups and/or one or more heteroatoms, such as oxygen, sulphur or nitrogen atoms, and may possibly be substituted by one or more hydroxyl or C₁- to C₈-alkoxy radicals, or a direct bond,
- G⁵ and G⁶, independently of one another, are a hydrogen or halogen atom, a C₁- to C₄-alkyl radical, a C₁- to C₄-monohydroxyalkyl radical, a C₂- to C₄-polyhydroxyalkyl radical, a C₁- to C₄-aminoalkyl radical or a direct bond to the bridge Y,
- G⁷, G⁸, G⁹, G¹⁰, G¹¹ and G¹², independently of one another, are a hydrogen atom, a direct bond to the bridge Y or a C₁- to C₄-alkyl radical,
with the provisos that
- the compounds of the formula (I) comprise only one bridge Y per molecule and
- the compounds of the formula (I) comprise at least one amino group which carries at least one hydrogen atom.

4. Agent according to Claim 3, **characterized in that** the compound of the formula (I) is chosen from N,N'-bis(β-hydroxyethyl)-N,N'-bis(4'-aminophenyl)-1,3-diaminopropan-2-ol, N,N'-bis(β-hydroxyethyl)-N,N'-bis(4'-aminophenyl)ethylenediamine, N,N'-bis(4-aminophenyl)tetramethylenediamine, N,N'-bis(β-hydroxyethyl)-N,N'-bis(4-aminophenyl)tetramethylenediamine, N,N'-bis(4-methylaminophenyl)tetramethylenediamine, N,N'-diethyl-N,N'-bis(4'-amino-3'-methylphenyl)ethylenediamine, bis(2-hydroxy-5-aminophenyl)methane, N,N'-bis(4'-aminophenyl)-1,4-diazacycloheptane, N,N'-bis(2-hydroxy-5-aminobenzyl)piperazine, N-(4'-aminophenyl)-p-phenylenediamine and 1,10-bis(2',5'-diaminophenyl)-1,4,7,10-tetraoxadecane and their physiologically compatible salts.

5. Agent according to Claim 4, **characterized in that** the compound of the formula (I) is chosen from N,N'-bis(β-hydroxyethyl)-N,N'-bis(4'aminophenyl)-1,3,-diaminopropan-2-ol, bis(2-hydroxy-5-aminophenyl)methane, N,N'-bis(4'-aminophenyl)-1,4-diazacycloheptane and 1,10-bis(2',5'-diaminophenyl)-1,4,7,10-tetraoxadecane and the physiologically compatible salts.

6. Agent according to one of Claims 1 to 5, **characterized in that** it further comprises at least one pyrimidine derivative.

7. Agent according to Claim 6, **characterized in that** the pyrimidine derivative is chosen from the group 2,4,5,6-tetraaminopyrimidine, 4-hydroxy-2,5,6-triaminopyrimidine and 2-hydroxy-4,5,6-triaminopyrimidine, and physiologically compatible salts thereof.

8. Agent according to one of Claims 1 to 7, **characterized in that** it comprises at least one further oxidation dye precursor of the developer or coupler type.

9. Agent according to one of Claims 1 to 8, **characterized in that** it further comprises at least one dye precursor of the indole or indoline type.

10. Agent according to one of Claims 1 to 9, **characterized in that** it further comprises at least one direct dye.

11. Method of dyeing keratin fibres, in particular hair, **characterized in that** an oxidation dye according to one of Claims 1 to 10, together with an oxidizing agent and/or with a catalyst for activating the oxidation, is applied to the fibres and, after a contact time, is rinsed out again with water or an aqueous surfactant preparation.

12. Use of oxidation dyes according to one of Claims 1 to 10 for dyeing keratin fibres, in particular human hair.

## Revendications

1. Teintures d'oxydation pour la teinture de fibres kératiniques, en particulier de cheveux, qui contiennent, dans un support aqueux
(A) comme composant de couplage au moins un 5-halogéno-2,4-diamino-alkylbenzène et
(B) comme composants de développement au moins un composant de développement à deux noyaux.

2. Teinture d'oxydation selon la revendication 1, **caractérisée en ce que** le 5-halogéno-2,4-diamino-alkylbenzène agissant comme agent de couplage est le 5-chloro-2,4-diaminotoluène.

3. Agent selon l'une quelconque des revendications 1 ou 2, **caractérisé en ce que** le composant de développement à deux noyaux est un composé de formule (I) 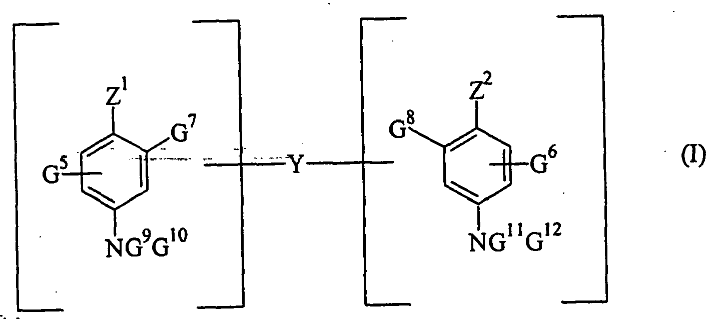 où:
- Z¹ et Z² représentent, indépendamment l'un de l'autre, un radical hydroxyle ou NH₂, qui est le cas échéant substitué par un radical alkyle en C₁ à C₄, par un radical hydroxyalkyle en C₁ à C₄ et/ou par un pont Y ou qui est le cas échéant une partie d'un système cyclique formant un pont,
- le pont Y représente un groupe alkylène comprenant 1 à 14 atomes de carbone, tel que par exemple une chaîne alkylène linéaire ou ramifiée ou un cycle alkylène, qui peut être interrompu ou terminé par un ou plusieurs groupes contenant de l'azote et/ou un ou plusieurs hétéroatomes tels que les atomes d'oxygène, de soufre ou d'azote et qui peut éventuellement être substitué par un ou plusieurs radicaux hydroxyle ou alcoxy en C₁ à C₈ ou une liaison directe,
- G⁵ et G⁶ représentent, indépendamment l'un de l'autre, un atome d'hydrogène ou d'halogène, un radical alkyle en C₁ à C₄, un radical monohydroxyalkyle en C₁ à C₄, un radical polyhydroxyalkyle en C₂ à C₄, un radical aminoalkyle en C₁ à C₄ ou une liaison directe avec le pont Y,
- G⁷, G⁸, G⁹, G¹⁰, G¹¹ et G¹² représentent, indépendamment l'un de l'autre, un atome d'hydrogène, une liaison directe de pontage Y ou un radical alkyle en C₁ à C₄,
à condition
- que les composés de formule (I) ne contiennent qu'un pont Y par molécule et
- que les composés de formule (1) contiennent au moins un groupe amino, qui porte au moins un atome d'hydrogène.

4. Agent selon la revendication 3, **caractérisé en ce que** le composé de formule (I) est choisi parmi le N,N'-bis-(β-hydroxyéthyl)-N,N'-bis-(4'-aminophényl)-1,3-diaminopropan-2-ol, la N,N'-bis-(β-hydroxyéthyl)-N,N'-bis-(4'-aminophényl)-éthylènediamine, la N,N'-bis-(4-aminophényl)-tétraméthylènediamine, la N,N'-bis-(β-hydroxyéthyl)-N,N'-bis-(4-aminophényl)-tétraméthylènediamine, la N,N'-bis-(4-méthylaminophényl)-tétraméthylènediamine, la N,N'-diéthyl-N,N'-bis-(4'-amino-3'-méthylphényl)-éthylènediamine, le bis-(2-hydroxy-5-aminophényl)-méthane, le N,N'-bis-(4'-aminophényl)-1,4-diazacycloheptane, la N,N'-bis-(2-hydroxy-5-aminobenzyl)-pipérazine, la N-(4'-aminophényl)-p-phénylènediamine et la 1,10-bis-(2',5'-diaminophényl)-1,4,7,10-tétraoxadécane et leurs sels physiologiquement acceptables.

5. Agent selon la revendication 4, **caractérisé en ce que** le composé de formule (1) est choisi parmi le N,N'-Bis-(β-hydroxyéthyl)-N,N'-bis-(4'-aminophényl)-1,3-diaminopropan-2-ol, le bis-(2-hydroxy-5-aminophényl)-méthane, le N,N'-bis-(4'-aminophényl)-1,4-diazacycloheptane et le 1,10-bis-(2',5'-diaminophényl)-1,4,7,10-tétraoxadécane et leurs sels physiologiquement acceptables.

6. Agent selon l'une quelconque des revendications 1 à 5, **caractérisé en ce qu'**il contient en outre au moins un dérivé de pyrimidine.

7. Agent selon la revendication 6, **caractérisé en ce que** le dérivé de pyrimidine est choisi dans le groupe formé par la 2,4,5,6-tétraaminopyrimidine, la 4-hydroxy-2,5,6-triaminopyrimidine et la 2-hydroxy-4,5,6-triaminopyrimidine ainsi que leurs sels physiologiquement acceptables.

8. Agent selon l'une quelconque des revendications 1 à 7, **caractérisé en ce qu'**il contient au moins un autre précurseur de colorant d'oxydation du type agent de développement ou de couplage.

9. Agent selon l'une quelconque des revendications 1 à 8, **caractérisé en ce qu'**il contient en outre au moins un autre précurseur de colorant de type indole ou indoline.

10. Agent selon l'une quelconque des revendications 1 à 9, **caractérisé en ce qu'**il contient en outre au moins un colorant montant directement sur la fibre.

11. Procédé pour la teinture de fibres kératiniques, en particulier de cheveux, **caractérisé en ce qu'**on applique sur les fibres un agent de teinture par oxydation selon l'une quelconque des revendications 1 à 10 avec un oxydant et/ou un catalyseur pour l'activation de l'oxydation et on l'élimine à nouveau par rinçage après un temps d'action avec de l'eau ou une préparation aqueuse tensioactive.

12. Utilisation d'agents de teinture d'oxydation selon l'une quelconque des revendications 1 à 10 pour la teinture de fibres kératiniques, en particulier de cheveux humains.
